(19) Europäisches Patentamt / European Patent Office / Office européen des brevets

(11) **EP 2 095 714 B1**

(12) **EUROPEAN PATENT SPECIFICATION**

(45) Date of publication and mention
of the grant of the patent:
**16.09.2020   Bulletin 2020/38**

(51) Int Cl.:
*A01N 37/16* (2006.01)       *A01N 25/02* (2006.01)
*A61L 2/18* (2006.01)          *C11D 3/39* (2006.01)
*A01P 1/00* (2006.01)

(21) Application number: **09153618.5**

(22) Date of filing: **25.02.2009**

(54) **Disinfecting and cold-sterilizing solution**

Desinfektions- und Kaltsterilisationslösung

Solution désinfectante et stérilisante à froid

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **26.02.2008   IT PD20080066**

(43) Date of publication of application:
**02.09.2009   Bulletin 2009/36**

(73) Proprietor: **Mondial S.N.C. di Cavinato Antonio &
C.
35010 Limena (PD) (IT)**

(72) Inventors:
 • **Cavinato, Antonio**
   **35010, LIMENA   PD (IT)**
 • **Cavinato, Lorenzo**
   **35010, LIMENA   PD (IT)**
 • **Cavinato, Roberto**
   **35010, LIMENA   PD (IT)**
 • **Cavinato, Gianantonio**
   **35010, LIMENA   PD (IT)**
 • **Cingano, Antonia**
   **35010, LIMENA   PD (IT)**

(74) Representative: **Modiano, Micaela Nadia
Modiano & Partners
Via Meravigli, 16
20123 Milano (IT)**

(56) References cited:
**EP-A1- 0 953 283          EP-A1- 2 180 786
EP-A2- 1 371 643           WO-A1-2006/016145
DE-A1- 3 615 787           US-A- 3 979 313
US-A1- 2008 045 593**

• **JÖRG HOFMANN ET AL: "Bleaching Activators
and the mechanism of bleaching activation",
JOURNAL FÜR PRAKTISCHE
CHEMIE/CHEMIKER-ZEITUNG, vol. 334, no. 4, 1
January 1992 (1992-01-01), pages 293-297,
XP055060838, ISSN: 0941-1216, DOI:
10.1002/prac.19923340402**
• **GUO JUN KANG ET AL: "Formation of peracetic
acid from hydrogen peroxide and pentaacetyl
glucose to activate oxygen delignification",
TAPPI JOURNAL, vol. 3, no. 1, 1 January 2002
(2002-01-01) , pages 19-22, XP055060842,**

**Description**

The present invention relates to a disinfecting and sterilizing composition having two components, in which the active ingredient is peracetic acid, to a method for preparing such disinfecting composition, and to a kit suitable to perform such method.

A primary need for many non-disposable materials, devices and instruments is that they must be sterilizable easily and effectively.

Among the methods currently most widespread for this purpose, there is the use of sterilization in an autoclave with moist or dry heat, a treatment which however is unsuitable for thermolabile devices.

It is further necessary to take into account the fact that many potentially effective sterilization methods are not usable because they do not have the necessary compatibility with the materials with which they come into contact.

Another drawback is the possible generation of substances that are dangerous for the operators and for the environment as a consequence of the sterilization activity.

In view of these problems, disinfecting solutions have been studied which are based on peracetic acid, a known disinfectant, which in relatively low concentrations is effective when used cold on almost all the materials with which it comes into contact without creating problems for the operators and the environment.

In order to keep diluted solutions of peracetic acid stable, however, they must be kept at a pH below 4, an aspect which creates additional problems due to the corrosive nature of the resulting solution.

For these reasons, the market has oriented itself toward diluted solutions of peracetic acid prepared extemporaneously so as to avoid the problems of long-term stability. Examples of solutions of this type are disclosed in EP 953283 and IT 1334939. These documents disclose multicomponent products that allow to obtain the disinfectant solution by mixing the various components at the time of use.

In particular, EP 953283 discloses a method for preparing the disinfectant based on peracetic acid by means of a reaction of a hydroalcoholic solution having acid pH composed of glacial or concentrated acetic acid and/or tetraacetyl ethylene diamine (TAED) or N,N',N",N'"-tetra acetyl glycol uril (TAGU), with a peroxide activator. The reaction between the hydroalcoholic solution and the activator generates the disinfecting active ingredient peracetic acid. Although effective, the product obtained from the mixing of the particular substances provided in the two components according to EP 953283 is characterized by a pungent odor, which despite being an indicator for the operator that mixing has occurred, can be unpleasant for the professional worker who is exposed every day to products of the type discussed here, making use of the product in the sanitary field more difficult.

EP1371643 discloses a system for a fast-rate preparation just before use of organic peroxyacids, in a hydroalcoholic environment, by mixing at temperatures lower than 40 °C, preferably lower than 25 °C, a first reactant and a second reactant, kept separate from each other during storage, to obtain an activated solution.

US3979313 discloses bleaching composition comprising a hydrogen peroxide adduct.

DE3615787 discloses a disinfectant, in particular for surfaces, instruments and clothing, based on oxygen donors and acyl donors, using as oxygen donor magnesium monoperoxyphthalate, where appropriate mixed with one or more other oxygen donors, and as acyl donor a mixture of two or three of the acetyl donors tetraacetylethylenediamine, pentaacetylglucose and tetraacetylglycoluril.

EP2180786 discloses shelf stable and/or less corrosive peroxycarboxylic acid antimicrobial compositions, including ready-to-use compositions

The aim of the present invention is to provide a disinfecting and sterilizing solution that allows to obtain easily a highly cold-sterilizing effect and at the most desired time, which maintains over time the stability both of its individual components and of the final solution, a solution whose pH is not aggressive for the materials with which it comes into contact and is not dangerous for operators assigned to preparing it, and which does not entail the formation of potentially dangerous compounds and does not leave residues on the substrates on which it is made to act.

Within this aim, an object of the invention is to provide a solution that has the necessary compatibility with all of the instruments, devices, surfaces and substrates that need to be sterilized.

Another object is to provide a solution as above that has high stability and is ready for use, both before its use and at the time of said use, so as to allow significant elasticity in the practical organization of sterilization operations.

Another object of the present invention is to provide a disinfecting and sterilizing composition that does not generate a pungent and unpleasant odor when it is used.

Another object of the invention is to provide a disinfecting and sterilizing composition that is highly reliable, relatively easy to provide and at competitive costs.

This aim and these and other objects that will become better apparent hereinafter, are achieved by a disinfecting and cold-sterilizing composition, characterized in that it has a buffered pH comprised between 4 and 8, and comprises at least two components, wherein the first component comprises hydrogen peroxide and the second component comprises glucose pentaacetate and a solvent selected from the group consisting of acetone, an ester of a mono- or polycarboxylic organic acid, dimethyl carbonate and mixtures thereof.

The aim and objects of the invention are also achieved by a kit for preparing a disinfecting and cold-sterilizing composition having a buffered pH comprised between 4 and 8, said kit being characterized in that it comprises two physically separate and non-connected compartments, wherein:

- the first compartment comprises a first component which comprises hydrogen peroxide;
- the second compartment comprises a second component which comprises glucose pentaacetate and a solvent selected from the group consisting of acetone, an ester of a mono- or poly- carboxylic organic acid, dimethyl carbonate and mixtures thereof.

Further, the aim and objects of the invention are also achieved by a method for preparing a disinfecting and cold-sterilizing solution with a buffered pH comprised between 4 and 8, comprising the step of mixing a first component that comprises hydrogen peroxide and a second component that comprises glucose pentaacetate and a solvent selected from the group consisting of acetone, an ester of a mono- or poly- carboxylic organic acid, dimethyl carbonate and mixtures thereof.

Further advantages of the invention will become better apparent in the continuation of the following detailed description.

Some of the characteristics of the invention will be presented hereafter in relation to the individual aspects of said invention.

In a first aspect, the present invention relates to a disinfecting and cold-sterilizing composition that performs its activity at a pH close to neutrality (between pH 4 and 8) and comprises at least two components, which are initially completely separated and from the reaction of which peracetic acid, the active ingredient of the composition, is obtained.

The first component comprises hydrogen peroxide, and in one embodiment of the invention such first component can be in the form of an aqueous solution. As an alternative, the first component can also be solid.

Preferably, the first component may comprise hydrogen peroxide in a quantity between 0.5% and 35% by weight on the weight of the first component, more preferably between 1% and 6% by weight, even more preferably equal to 5% by weight.

In one embodiment of the present invention, the first component may be characterized by a high concentration of hydrogen peroxide, preferably between 10% and 30% by weight on the weight of the first component, more preferably equal to 20% by weight, from the dilution of which, prior to mixing with the second component, a more diluted solution of the first component is obtained. Advantageously, this embodiment allows to avoid resorting to large volumes, with positive consequences in terms of logistics and costs related to storage, transport and packaging.

In another embodiment, the first component may be characterized by a lower concentration of hydrogen peroxide, particularly 1% to 6% by weight, more preferably 5% by weight, so as to be already diluted. Advantageously, this embodiment leads to having a solution that is ready for use to prepare the disinfecting composition according to the invention.

The second component comprises glucose pentaacetate (GPA) and a solvent selected from the group consisting of acetone, an ester of a mono- or poly- carboxylic organic acid, dimethyl carbonate and mixtures thereof.

GPA is an acetic ester without reducing properties, which generally is prepared by hot reaction of glucose ($C_6H_{12}O_6$) with acetic anhydride ($C_4H_6O_3$) in the presence of a zinc chloride ($ZnCl_2$) catalyst. GPA is soluble in solvents such as alkyl ethers of ethylenic or propylenic glycols, aliphatic ketones, esters of carboxylic acids, aliphatic carbonates and mixtures thereof, and is scarcely soluble in alcoholic solvents such as for example ethanol (GPA solubility lower than 5% by weight) and isopropanol (GPA solubility lower than 3% by weight).

The absence of reducing properties demonstrates that GPA is a derivative of the hemiacetal form, and not of the aldehyde form, in which the glucosidic hydroxyl also has undergone acetylation. There are therefore two isomeric acetyl derivatives that correspond to the two methyl glucosides $\alpha$ and $\beta$. By hydrolysis it is possible to separate all the acetyl groups, except for the one joined to the glucosidic hydroxyl, and therefore GPA can be defined as a precursor of peracetic acid, since it is a donor of acetyl groups. Advantageously, GPA, thanks to the presence of multiple acetyl groups, is configured as a precursor of peracetic acid that is extremely effective, since under hydrolytic conditions it makes available up to four acetyl groups from which the disinfecting active ingredient can form.

Further, the final product of the hydrolysis reaction of the acetyl groups of GPA is a derivative of glucose, whereas in the case of a donor of acetyl groups such as tetracetyl ethylene diamine (TAED) the final product is represented by ethylene diamine, a compound characterized by a corrosive action and an irritant by contact with the skin or inhalation. Accordingly, GPA has the additional advantage of being converted into a final product that is harmless for the operator even in case of repeated contact with the skin or inhalation.

According to the invention, the solvent is selected from the group consisting of acetone, an ester of a mono- or poly- carboxylic organic acid, dimethyl carbonate, and mixtures thereof.

Further, in another embodiment of the present invention, the second component may be anhydrous.

Preferably, the second component may comprise glucose pentaacetate in a quantity between 2% and 30% by weight on the weight of the second component, more preferably 4% to 15% by weight, and the solvent in a quantity up to 98% by weight on the weight of the second component, more preferably up to 95% by weight.

Surprisingly, it has been observed that the disinfecting composition, in which the second component comprises GPA and one or more of said solvents, lacks the characteristic pungent odor that is typical of a disinfecting composition based on peracetic acid, as disclosed for example in EP 953283.

In another embodiment, the second component may further comprise a second solvent selected from the group consisting of propylene glycol monomethyl ether, dipropylene glycol monomethyl ether, propylene carbonate and mixtures thereof.

In another embodiment, the composition according to the invention may be characterized in that the first component further comprises at least one buffer system.

The expression "buffer system" is used to reference a conjugate acid-base pair with a pK value (negative logarithm of the dissociation constant) that is close to neutrality or a compound which, in the presence of water, generates a conjugate acid-base pair with a pK value that is proximate to neutrality, wherein the acid-base pair is capable of absorbing pH variations, keeping the total pH of the disinfecting solution at a value that is close to neutrality, advantageously in an interval comprised between 4 and 8.

Preferably, the buffer system may comprise a buffering agent that is selected from the group consisting of a weak organic base, a conjugate base of a mono- or poly- protic weak organic acid, an organic salt, and mixtures thereof. Preferably, the organic salt is sodium acetate.

For example, the buffer system may be an aqueous solution of sodium acetate, where the sodium acetate may be in a quantity between 0.1% and 20% by weight on the weight of the first component, preferably from 0.3% to 1% by weight.

In another embodiment, the first component of the composition may further comprise at least one stabilizing system.

The expression "stabilizing system" is used to reference a compound that is capable of applying a chelating or sequestering action to a substrate, usually a metallic ion, by forming a complex with said substrate.

Preferably, the stabilizing system may comprise a stabilizing agent selected from the group consisting of a diphosphonic acid, ethylenediaminetetraacetic acid (EDTA), sodium pyrophosphate and a sodium salt of an aminoalkylphosphonate acid. Even more preferably, the stabilizing agent may be a sodium salt of an aminoalkylphosphonate acid.

For example, the stabilizing system may be an aqueous solution of a sodium salt of an aminoalkylphosphonate acid in a quantity between 0.01% and 1% by weight on the weight of the first component, preferably between 0.05% and 0.3% by weight.

Of course, such stabilizing agents may be replaced with any agent having a metallic ion sequestrant or chelating function.

In another embodiment, the first component of the composition may further comprise at least one corrosion inhibiting system.

The expression "corrosion inhibiting system" is used to reference a compound that is capable of inducing chemical reactions that are parallel or competitive with respect to the corrosion process, preventing for a certain period of time the corrosion of the material in contact with such system.

Preferably, the corrosion inhibiting system may comprise a corrosion inhibitor that is selected from the group consisting of a heterocyclic condensed ring compound, such as for example benzotriazole, tolyltriazole or mercaptobenzothiazole, a sodium salt of a condensed ring heterocyclic compound, a 5-atom cyclic compound, an azole, a benzoate, a phosphate and derivatives thereof, a borate, a chromate, a dichromate, a tungstate, a vanadate and combinations of these compounds. More preferably, the corrosion inhibiting system may be a heterocyclic condensed ring compound.

For example, the corrosion inhibiting system may be an aqueous solution of a heterocyclic condensed ring compound in a quantity from 0.01% to 2% by weight on the weight of the first component, preferably from 0.05% to 0.2% by weight.

In another embodiment, the first component of the composition may further comprise at least one surfactant system.

The expression "surfactant system" is used to reference a compound that is capable of reducing surface tension and allowing more easily the removal of residues that are present on the surfaces in contact with such system.

Preferably, the surfactant system may comprise a surfactant selected from the group consisting of an anionic surfactant, a cationic surfactant, a non-ionic surfactant, an amphoteric surfactant and mixtures thereof.

For example, the anionic surfactant may be selected from the group consisting of sulfonated fatty acids and fatty acid sulphates, the cationic surfactant may be a quaternary ammonium compound, and the non-ionic surfactant may be an alkyl polyglucoside.

In a preferred embodiment, the surfactant system may be an aqueous solution of one or more of the above mentioned surfactants, in a quantity from 0.01% to 2% by weight on the weight of the first component, preferably from 0.01% to 0.5% by weight.

In a second aspect, the present invention consists of a kit for preparing a disinfecting and sterilizing composition as described here starting from the first component comprising hydrogen peroxide and the second component comprising GPA and a solvent selected from the group consisting of acetone, an ester of a mono- or poly- carboxylic organic acid, dimethyl carbonate and mixtures thereof. This kit provides for the presence of two separate compartments, which are not directly connected and within which the two components are kept completely separate from each other. When the two components are in the same environment, peracetic acid is generated and provides the disinfecting action of the composition.

In one embodiment of the kit according to the invention, the first component may be in the form of an aqueous solution. In another embodiment of the kit, the first component may comprise hydrogen peroxide in a quantity between 0.5% and 35% by weight on the weight of the first composition.

In another embodiment of the kit according to the invention, the second component may comprise GPA in a quantity from 2% to 30% by weight on the weight of the second component and the solvent in a quantity up to 98% by weight on the weight of the second component.

In a further aspect, the invention also relates to a method for on-demand preparation of a disinfecting and cold-sterilizing composition with a buffered pH comprised between 4 and 8, said method comprising the step of mixing a first component that comprises hydrogen peroxide and a second component that comprises glucose pentaacetate and a solvent selected from the group consisting of acetone, an ester of a mono- or poly- carboxylic organic acid, dimethyl carbonate and mixtures thereof..

There is no predefined and immutable order according to which the two components are to be mixed. It is possible to proceed by mixing the first component in the second one, obtaining a mixture of GPA, solvent and hydrogen peroxide, and likewise it is also possible to mix the second component in the first one. In a preferred embodiment, the second component is added to the first component.

Preferably, in the method according to the invention the first component may comprise hydrogen peroxide in a quantity from 0.5% to 35% by weight on the weight of the first component.

The Applicant has in fact found that at least some of the solvents listed above and their mixtures exhibit, even in the absence of water as a cosolvent, a high solvent power for GPA, which allows to provide the second component in a highly concentrated form without giving rise to (re)precipitation phenomena. Said solvents therefore maximize the high addition of acetate that is characteristic of GPA, with a consequent considerable saving in volume occupation of the respective containers.

In another embodiment of the method according to the invention, a further phase may be included which is adapted to prepare the first component prior to the step for mixing the first and second components. Such additional step may be performed by choosing among the following methods:

- diluting, preferably in water, a concentrated hydrogen peroxide solution, and
- dissolving, preferably in water, at least one agent selected among urea peroxide, percarbonates, perborates, persulfates and tert-butyl hydroperoxide.

Further characteristics and advantages of the invention will become better apparent from the following detailed description of a preferred but not exclusive embodiment of the composition and of the method according to the invention, illustrated by way of non-limiting indication in the examples that follow. In these examples, the first component comprising hydrogen peroxide is identified as component A, whereas the second component comprising GPA and a solvent selected from the group consisting of acetone, an ester of a mono- or poly- carboxylic organic acid, dimethyl carbonate and mixtures thereof, is identified as component B.

Example 1

A solution constituting component B and having the following composition was prepared:

| Component | % by wt. | g/50 g solution |
|---|---|---|
| Glucose pentaacetate | 10 | 5.0 |
| Dimethyl carbonate | 90 | 45.0 |

The following instruments were used for preparation:

- 250-ml and 1000-ml containers

- a magnetic agitator

- a Mettler Toledo - Viper BC technical balance.

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | $1.068 \pm 0.010$ |
| Appearance | | Clear |
| Color | | Colorless |

50 g of component B comprising the components described above were added to 550 g of component A, preferably composed as follows (where $H_2O_2$ 35% in $H_2O$ identifies a 35% hydrogen peroxide solution in water):

| Component | % by wt. | g/550 g of solution |
|---|---|---|
| $H_2O_2$ 35% in $H_2O$ | 14 | 77.0 |
| Distilled water | 85.25 | 468.9 |
| Stabilizing agent | 0.1 | 0.5 |
| Sodium acetate | 0.5 | 2.7 |
| Corrosion inhibitor | 0.15 | 0.8 |
| *Density (20°C)* | g/ml | $1.020 \pm 0.010$ |

After mixing of the two components, the chemical and physical properties of the resulting solution were as follows:

| Property | Unit of measure | Value |
|---|---|---|
| Density (20°C) | g/ml | $1.025 \pm 0.010$ |
| pH (10% by volume in distilled water) | U of pH | $6.51 \pm 0.05$ |
| Appearance | | Clear |
| Color | | Colorless |

Example 2

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Dimethyl carbonate | 24 | 12.0 |
| Dipropylene glycol monomethyl ether | 66 | 33.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.015 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.027 ± 0.010 |
| pH (10% by volume in distilled water) | U of pH | 6.45 ± 0.05 |
| Appearance | | Clear |
| Color | | Colorless |

Example 3

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Dimethyl carbonate | 24 | 12.0 |
| Propylene glycol monomethyl ether | 66 | 33.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.990 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.020 ± 0.010 |
| pH (10% by volume in distilled water) | U of pH | 6.48 ± 0.05 |
| Appearance | | Clear |

(continued)

| Property | Unit of measure | Value |
|---|---|---|
| Color | | Colorless |

Example 4

The same procedure adopted and described in Example 3 was followed and a solution constituting component B and having the following composition was prepared:

| Component | % by wt. | g/50 g solution |
|---|---|---|
| Glucose pentaacetate | 10 | 5.0 |
| Propylene glycol monomethyl ether | 46 | 23.0 |
| Dipropylene glycol monomethyl ether | 20 | 10.0 |
| Dimethyl carbonate | 24 | 12.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $0.987 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.026 \pm 0.010$ |
| pH (10% by volume in distilled water) | U of pH | $6.45 \pm 0.05$ |
| Appearance | | Clear |
| Color | | Colorless |

Example 5

The same procedure adopted and described in Example 3 was followed and a solution constituting component B and having the following composition was prepared:

| Component | % by wt. | g/50 g solution |
|---|---|---|
| Glucose pentaacetate | 10 | 2.5 |
| Propylene glycol monomethyl ether | 20 | 10.0 |
| Dipropylene glycol monomethyl ether | 46 | 23.0 |
| Dimethyl carbonate | 24 | 12.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.000 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.028 ± 0.010 |
| pH (10% by volume in distilled water) | U of pH | 6.52 ± 0.05 |
| Appearance | | Clear |
| Color | | Colorless |

Example 6

The same procedure adopted and described in Example 1 was followed and a solution constituting component B and having the following composition was prepared:

| Component | % by wt. | g/50 g solution |
|---|---|---|
| Glucose pentaacetate | 12 | 6.0 |
| Acetone | 88 | 44.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.800 ± 0.010 |
| Appearance | | Clear |
| Color | | Colorless |

50 g of component B comprising the components described above were added to 550 g of component A.

The composition of component A was the one described in Example 1.

After mixing of the two components, the chemical and physical properties of the resulting solution were as follows:

| Property | Unit of measure | Value |
|---|---|---|
| Density (20°C) | g/ml | 1.010 ± 0.010 |
| pH (10% by volume in distilled water) | U of pH | 6.43 ± 0.05 |

(continued)

| Property | Unit of measure | Value |
|---|---|---|
| Appearance | | Clear |
| Color | | Colorless |

Example 7

The same procedure adopted and described in Example 6 was followed and a solution constituting component B and having the following composition was prepared:

| Component | % by wt. | g/50 g solution |
|---|---|---|
| Glucose pentaacetate | 12 | 6.0 |
| Acetone | 30 | 15.0 |
| Propylene glycol monomethyl ether | 58 | 29.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $1.020 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.020 \pm 0.010$ |
| pH (10% by volume in distilled water) | U of pH | $6.53 \pm 0.05$ |
| Appearance | | Clear |
| Color | | Colorless |

Example 8

The same procedure adopted and described in Example 6 was followed and a solution constituting component B and having the following composition was prepared:

| Component | % by wt. | g/50 g solution |
|---|---|---|
| Glucose pentaacetate | 10 | 5.0 |
| Dimethyl carbonate | 24 | 12.0 |
| Acetone | 66 | 33.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.910 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.023 ± 0.010 |
| pH (10% by volume in distilled water) | U of pH | 6.53 ± 0.05 |
| Appearance | | Clear |
| Color | | Colorless |

Example 9

The same procedure adopted and described in Example 6 was followed and a solution constituting component B and having the following composition was prepared:

| Component | % by wt. | g/50 g solution |
|---|---|---|
| Glucose pentaacetate | 12 | 6.0 |
| Acetone | 30 | 15.0 |
| Dipropylene glycol monomethyl ether | 58 | 29.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.920 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.022 ± 0.010 |

(continued)

| Property | Unit of measure | Value |
|---|---|---|
| pH (10% by volume in distilled water) | U of pH | 6.47 ± 0.05 |
| Appearance | | Clear |
| Color | | Colorless |

Example 10

The same procedure adopted and described in Example 7 was followed and a solution constituting component B and having the following composition was prepared:

| Component | % by wt. | g/50 g solution |
|---|---|---|
| Glucose pentaacetate | 5 | 2.5 |
| Propylene glycol monomethyl ether | 65 | 32.5 |
| Dipropylene glycol monomethyl ether | 20 | 10.0 |
| Acetone | 10 | 5.0 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | 0.950 ± 0.010 |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | 1.026 ± 0.010 |
| pH (10% by volume in distilled water) | U of pH | 6.49 ± 0.05 |
| Appearance | | Clear |
| Color | | Colorless |

Example 11 (not according to the invention as claimed)

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Butyl diglycol acetate | 95 | 47.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.000 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.000 ± 0.010 |
| pH (10% by volume in distilled water) | U of pH | 6.50 ± 0.05 |
| Appearance | | Clear |
| Color | | Colorless |

Example 12

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Dimethyl glutarate | 95 | 47.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.080 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.080 ± 0.010 |
| pH (10% by volume in distilled water) | U of pH | 6.55 ± 0.05 |

(continued)

| Property | Unit of measure | Value |
|---|---|---|
| Appearance | | Clear |
| Color | | Colorless |

Example 13

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Ethyl acetate | 95 | 47.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $0.9200 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.000 \pm 0.010$ |
| pH (10% by volume in distilled water) | U of pH | $6.52 \pm 0.05$ |
| Appearance | | Clear |
| Color | | Colorless |

Example 14

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Ethyl acetate | 90 | 45 |
| Dimethyl succinate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.920 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.000 ± 0.010 |
| pH (10% by volume in distilled water) | U of pH | 6.45 ± 0.05 |
| Appearance | | Clear |
| Color | | Colorless |

Example 15

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Ethyl acetate | 92.5 | 46.25 |
| Dimethyl glutarate | 2.5 | 1.25 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.920 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.000 ± 0.010 |
| pH (10% by volume in distilled water) | U of pH | 6.40 ± 0.05 |

(continued)

| Property | Unit of measure | Value |
|---|---|---|
| Appearance | | Clear |
| Color | | Colorless |

Example 16

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Propylene carbonate | 90 | 45 |
| Dimethyl succinate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $1.090 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.045 \pm 0.010$ |
| pH (10% by volume in distilled water) | U of pH | $6.50 \pm 0.05$ |
| Appearance | | Clear |
| Color | | Colorless |

Example 17

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Dipropylene glycol monomethyl ether | 90 | 45 |
| Dimethyl glutarate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.015 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.012 ± 0.010 |
| pH (10% by volume in distilled water) | U of pH | 6.45 ± 0.05 |
| Appearance | | Clear |
| Color | | Colorless |

Example 18

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Propylene glycol monomethyl ether | 80 | 40 |
| Dipropylene glycol monomethyl ether | 10 | 5 |
| Dimethyl succinate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.950 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | $1.010 \pm 0.010$ |
| pH (10% by volume in distilled water) | U of pH | $6.56 \pm 0.05$ |
| Appearance | | Clear |
| Color | | Colorless |

Example 19

The same procedure adopted and described in Example 1 was followed and a solution constituting component B and having the following composition was prepared:

| Component | % by wt. | g/50 g solution |
|---|---|---|
| Glucose pentaacetate | 10 | 5 |
| Propylene glycol monomethyl ether | 40 | 20 |
| Ethyl acetate | 40 | 20 |
| Dimethyl adipate | 10 | 5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $0.955 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $0.9880 \pm 0.010$ |
| pH (10% by volume in distilled water) | U of pH | $6.48 \pm 0.05$ |
| Appearance | | Clear |
| Color | | Colorless |

Example 20

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Propylene glycol monomethyl ether | 50 | 25 |

(continued)

| Component | % by wt. | g/50 g solution |
|---|---|---|
| Dimethyl glutarate | 45 | 22.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 1.000 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.000 ± 0.010 |
| pH (10% by volume in distilled water) | U of pH | 6.46 ± 0.05 |
| Appearance | | Clear |
| Color | | Colorless |

Example 21

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Dipropylene glycol monomethyl ether | 40 | 20 |
| Ethyl acetate | 50 | 25 |
| Dimethyl succinate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.900 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
| --- | --- | --- |
| Density (20°C) | g/ml | 1.000 ± 0.010 |
| pH (10% by volume in distilled water) | U of pH | 6.52 ± 0.05 |
| Appearance | | Clear |
| Color | | Colorless |

### Example 22

The same procedure adopted and described in Example 1 was followed and a solution constituting component B and having the following composition was prepared:

| Component | % by wt. | g/50 g solution |
| --- | --- | --- |
| Glucose pentaacetate | 5 | 2.5 |
| Dipropylene glycol monomethyl ether | 45 | 22.5 |
| Ethyl acetate | 50 | 25 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
| --- | --- | --- |
| Density (20°C) | g/ml | 0.9400 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
| --- | --- | --- |
| Density (20°C) | g/ml | 0.9700 ± 0.010 |
| pH (10% by volume in distilled water) | U of pH | 6.46 ± 0.05 |
| Appearance | | Clear |
| Color | | Colorless |

### Example 23

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Propylene glycol | 20 | 10 |
| Ethyl acetate | 70 | 35 |
| Acetone | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.935 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.015 ± 0.010 |
| pH (10% by volume in distilled water) | U of pH | 6.35 ± 0.05 |
| Appearance | | Clear |
| Color | | Colorless |

Example 24

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Acetone | 90 | 45 |
| Dimethyl adipate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.9300 ± 0.010 |
| Appearance | | Clear |

(continued)

| Property | Unit of measure | Value |
|---|---|---|
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.015 ± 0.010 |
| pH (10% by volume in distilled water) | U of pH | 6.67 ± 0.05 |
| Appearance | | Clear |
| Color | | Colorless |

Example 25

The same procedure adopted and described in Example 1 was followed and a solution constituting component B and having the following composition was prepared:

| Component | % by wt. | g/50 g solution |
|---|---|---|
| Glucose pentaacetate | 5 | 2.5 |
| Dipropylene glycol monomethyl ether | 30 | 15 |
| Acetone | 60 | 30 |
| Dimethyl glutarate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.9700 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.000 ± 0.010 |
| pH (10% by volume in distilled water) | U of pH | 6.54 ± 0.05 |
| Appearance | | Clear |
| Color | | Colorless |

Example 26

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Ethyl acetate | 30 | 15.0 |
| Acetone | 55 | 27.5 |
| Dimethyl glutarate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $0.8561 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.000 \pm 0.010$ |
| pH (10% by volume in distilled water) | U of pH | $6.57 \pm 0.05$ |
| Appearance | | Clear |
| Color | | Colorless |

Example 27

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Ethyl acetate | 10 | 5.0 |
| Acetone | 77 | 38.5 |
| Dimethyl glutarate | 3 | 1.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | 0.8306 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.009 ± 0.010 |
| pH (10% by volume in distilled water) | U of pH | 6.59 ± 0.05 |
| Appearance | | Clear |
| Color | | Colorless |

Example 28

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Ethyl acetate | 10 | 5.0 |
| Acetone | 75 | 37.5 |
| Dimethyl glutarate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.8388 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.012 ± 0.010 |
| pH (10% by volume in distilled water) | U of pH | 6.61 ± 0.05 |
| Appearance | | Clear |

(continued)

| Property | Unit of measure | Value |
|---|---|---|
| Color | | Colorless |

Example 29

The same procedure adopted and described in Example 1 was followed and a solution constituting component B and having the following composition was prepared:

| Component | % by wt. | g/50 g solution |
|---|---|---|
| Glucose pentaacetate | 10 | 5.0 |
| Ethyl acetate | 5 | 2.5 |
| Acetone | 80 | 40 |
| Dimethyl glutarate | 5 | 2.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | $0.8315 \pm 0.010$ |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | $1.012 \pm 0.010$ |
| pH (10% by volume in distilled water) | U of pH | $6.60 \pm 0.05$ |
| Appearance | | Clear |
| Color | | Colorless |

Example 30

The same procedure adopted and described in Example 1 was followed and a solution constituting component B and having the following composition was prepared:

| Component | % by wt. | g/50 g solution |
|---|---|---|
| Glucose pentaacetate | 10 | 5 |
| Ethylene glycol diethyl ether | 10 | 5 |
| Acetone | 80 | 40 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.8286 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.009 ± 0.010 |
| pH (10% by volume in distilled water) | U of pH | 6.66 ± 0.05 |
| Appearance | | Clear |
| Color | | Colorless |

Example 31

The same procedure adopted and described in Example 1 was followed and a solution constituting component B and having the following composition was prepared:

| Component | % by wt. | g/50 g solution |
|---|---|---|
| Glucose pentaacetate | 10 | 5 |
| Ethylene glycol diethyl ether | 5 | 2.5 |
| Acetone | 75 | 37.5 |
| Ethyl acetate | 10 | 5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.

All the operations were performed at ambient temperature (20°C).

The following chemical and physical properties were measured:

| Property | Unit of measure | Value |
|---|---|---|
| Density (20°C) | g/ml | 0.8336 ± 0.010 |
| Appearance | | Clear |
| Color | | Colorless |

50 g of component B comprising the components described above were added to 550 g of component A. The composition of component A was the one described in Example 1.

After mixing of the two components, the chemical and physical properties of the resulting solution were as follows:

| Property | Unit of measure | Value |
|---|---|---|
| Density (20°C) | g/ml | 1.010 ± 0.010 |
| pH (10% by volume in distilled water) | U of pH | 6.59 ± 0.05 |
| Appearance | | Clear |
| Color | | Colorless |

Example 32

The same procedure adopted and described in Example 1 was followed and a solution constituting component B and having the following composition was prepared:

| Component | % by wt. | g/50 g solution |
|---|---|---|
| Glucose pentaacetate | 10 | 5 |
| Ethylene glycol diethyl ether | 1 | 0.5 |
| Acetone | 87 | 43.5 |
| Ethyl acetate | 1 | 0.5 |
| Dimethyl glutarate | 1 | 0.5 |

The listed components were weighed separately and mixed in the sequence described above. After the addition of all the components, the mixture was subjected to magnetic agitation for 10 minutes and left to rest for a further 10 minutes. A sample was then taken to check the chemical and physical characteristics of the preparation.
All the operations were performed at ambient temperature (20°C).
The following chemical and physical properties were measured:

| Property | Unit of measure | Value |
|---|---|---|
| Density (20°C) | g/ml | 0.8179 ± 0.010 |
| Appearance | | Clear |
| Color | | Colorless |

50 g of component B comprising the components described above were added to 550 g of component A. The composition of component A was the one described in Example 1.
After mixing of the two components, the chemical and physical properties of the resulting solution were as follows:

| Property | Unit of measure | Value |
|---|---|---|
| Density (20°C) | g/ml | 1.009 ± 0.010 |
| pH (10% by volume in distilled water) | U of pH | 6.45 ± 0.05 |
| Appearance | | Clear |
| Color | | Colorless |

It has been found that all compositions obtained from the mixing of components A and B according to Examples 1-32 lack the characteristic pungent odor that is typical of disinfectant compositions based on peracetic acid as disclosed for example in EP 953283.

Example 33

In order to check the effectiveness as a disinfectant of the composition according to the invention, compositions prepared as described in Example 6, Example 7 and Example 24 were subjected to a disinfecting effectiveness assessment according to the UNI EN 14561 standard.

The following bacterial strains, all purchased at the Pasteur Institute of Paris, were used: *Pseudomonas aeruginosa* (ATCC 15442), *Staphylococcus aureus* (ATCC 6538) and *Enterococcus hirae* (ATCC 10541). The bacterial strains were incubated on TSA (Tryptone Soya Agar) medium at 37°±1°C for 18 hours and bacterial suspensions having a concentration comprised between 1.5x10$^9$ and 5.0x10$^9$ ufc/ml were prepared for each bacterial strain, starting from the final cultures, within two hours of the beginning of the tests.

Two supports were prepared for each bacterial strain, with 0.05 ml of a mixture consisting of 9 ml of bacterial suspension and 1 ml of an aqueous solution comprising bovine albumin at a concentration of 0.03 g/1. The supports were allowed to dry at 37°±1°C for 1 hour and then placed in contact with 10 ml of each one of the compositions of Examples 6, 7 and 24 for 10 minutes at 20°±1°C.

At the end of the 10 minutes, the support were transferred in 10 ml of a neutralizing solution having the following formulation and subjected to agitation for 15 seconds:

| | |
|---|---|
| - lecithin | 3 g |
| - polysorbate 80 | 30 g |
| - sodium thiosulfate | 5 g |
| - L-histidine | 1 g |
| - saponin | 30 g |
| - tryptonated water | q.s. to 1000 ml. |

After a neutralization time equal to 5 minutes ± 10 seconds, the mixture was diluted by serial decimal dilutions from 10$^{-0}$ to 10$^{-3}$, and then volumes of 1 ml were drawn from the mixture and from its dilutions and placed in Petri dishes in double. All the dishes were incubated at 37°±1°C for 48 hours.

The entire procedure was repeated on additional supports inoculated with 0.05 ml of the mixture of bacterial suspension and albumin solution and by placing said additional supports in contact with water instead of the solutions of examples 6, 7 and 24.

After incubation of the Petri dishes, the number of ufc/dish was counted for each dilution, and the count of ufc/ml for the samples placed in contact with the solutions according to the invention (Na) and the count of ufc/ml for the samples placed in contact with water (Nw) were performed on the basis of the following formula:

$$ufc/ml = \frac{C}{n \, x \, V \, x \, d}$$

where C = sum of the colonies counted on the dishes being considered; n = number of counted dishes; V = used volume; and d = dilution factor corresponding to the dilution performed.

The disinfecting effectiveness of the compositions according to the invention was determined by calculating the reduction in bacterial vitality as a consequence of contact with the compositions according to the invention used in the tests by means of the following formula:

$$R = Log\,Nw - Log\,Na$$

The tested compositions were considered effective according to UNI EN 14561 when, for each bacterial strain, they produced a reduction in vitality of at least 5 log at 20°C within 60 minutes of contact.

The values of Na and Nw and R for each composition tested with respect to each bacterial strain are given in Table 1.

Table 1

| Solution | Bacterial strain | Na (ufc/ml) | Nw (ufc/ml) | R (Log ufc/ml) |
|---|---|---|---|---|
| Example 6 | *P. aeruginosa* | <2.15 | 7.97 | >5.82 |
| | *S. aureus* | <2.15 | 7.91 | >5.76 |
| | *E. hirae* | <2.15 | 7.58 | >5.43 |

(continued)

| Solution | Bacterial strain | Na (ufc/ml) | Nw (ufc/ml) | R (Log ufc/ml) |
|---|---|---|---|---|
| Example 7 | P. aeruginosa | <2.15 | 7.97 | >5.82 |
| | S. aureus | <2.15 | 7.91 | >5.76 |
| | E. hirae | <2.15 | 7.58 | >5.43 |
| Example 24 | P. aeruginosa | <2.15 | 7.97 | >5.82 |
| | S. aureus | <2.15 | 7.91 | >5.76 |
| | E. hirae | <2.15 | 7.58 | >5.43 |

It has thus been found that the composition according to the invention is characterized by an effective disinfectant property, capable of reducing the vitality of the microorganisms with which it comes into contact in a short period of time.

Further, it has also been observed that the composition, the kit and the method according to the invention fully achieve the intended aim, since the composition has a high oxidation potential for peracetic acid in equilibrium with the other components, has significant cold-biocidal and sporicidal properties, is sufficiently diluted to have no toxicity, has a nonaggressive pH which in any case is such as to be compatible with the most disparate types of materials to be sterilized, does not entail risks for operators, possesses the necessary stability to allow a certain elasticity in the periods of use and preservation, is easy to obtain and to dispose after use, and does not leave residues on the sterilized materials.

Moreover, it has been observed that the kit according to the invention, by segregating the fundamental components into independent compartments which are not connected directly, allows to obtain a system that is stable over time, eliminating the need, until now deemed essential, to keep at least one of the components at a highly acid pH. Due to the chemical homogeneity of each of the individual components, it is in fact possible to adopt specific stabilization conditions for each individual component, conditions which instead might be ineffective or even harmful for the other components.

Further, it has been observed that in the kit according to the invention, thanks to the high solvent power of the solvents of the second component, it is possible to vary the percentage of GPA that is present in the second component and the volume ratio between the two compartments, thus obtaining in a highly versatile manner solutions of peracetic acid in which the quantity of bactericidal active ingredient varies from 1300 ppm to 5000 ppm.

It has also been observed that the final sterilizing composition is capable of remaining stable and of having sufficient disinfectant properties for at least 14 days after its preparation.

Further, it has been observed that since the composition according to the invention lacks strongly acid components, the risks of danger for operators and for the environment are reduced further.

Further, it has been observed that the composition according to the invention entails a lower logistical management cost if a component that contains hydrogen peroxide in concentrated form is used.

Moreover, it has been observed that the composition according to the invention maintains a significant effectiveness over time even without resorting to different chemical types of acetylated compounds, such as for example a combination of N- and O-acetylated compounds.

Moreover, the composition according to the invention, by virtue of the solvents that constitute the second component, contributes to a higher effectiveness in cleaning the surfaces on which it is used. Moreover, such effectiveness is increased further if the composition comprises a surfactant system in the first component.

Finally, it has also been found that the composition according to the invention at the time of the mixing of the two components does not generate unpleasant and/or pungent odors that might be an inconvenience for sterilization workers.

**Claims**

1. A disinfecting and cold-sterilizing composition, having a buffered pH comprised between 4 and 8, and comprising at least a first component and a second component,
   wherein the first component comprises hydrogen peroxide and
   the second component comprises glucose pentaacetate and a solvent,
   wherein the solvent is selected from the group consisting of acetone, an ester of a mono- or poly- carboxylic organic acid, dimethyl carbonate and mixtures thereof.

2. The composition according to claim 1, wherein the second component further comprises a second solvent selected from the group consisting of propylene glycol monomethyl ether, dipropylene glycol monomethyl ether, propylene carbonate and mixtures thereof.

3. The composition according to claim 1, wherein the first component comprising hydrogen peroxide is in the form of an aqueous solution or in solid form.

4. The composition according to claim 1, wherein the first component comprises hydrogen peroxide in a quantity from 0.5% to 35% by weight on the weight of the first component.

5. The composition according to claim 1, wherein the second component comprises glucose pentaacetate in a quantity from 2% to 30% by weight on the weight of the second component and the solvent in a quantity up to 98% by weight on the weight of the second component.

6. The composition according to claim 1, wherein the second component is anhydrous.

7. The composition according to claim 1, wherein the first component further comprises at least one buffer system.

8. The composition according to claim 1, wherein the first component further comprises at least one stabilizing system.

9. The composition according to claim 8, wherein the at least one stabilizing system comprises a stabilizing agent selected from the group consisting of a diphosphonic acid, ethylenediaminetetraacetic acid (EDTA), sodium pyrophosphate and a sodium salt of an amino alkyl phosphonate acid.

10. The composition according to claim 1, wherein the first component further comprises at least one corrosion inhibiting system.

11. The composition according to claim 1, wherein the first component further comprises at least one surfactant system.

12. A kit for preparing a disinfecting and cold-sterilizing composition having a buffered pH comprised between 4 and 8 according to anyone of claims 1-11, said kit comprising two physically separate and non-connected compartments, wherein:

   - a first compartment of said compartments comprises said first component; and
   - a second compartment of said compartments comprises said second component.

13. A method for preparing a disinfecting and cold-sterilizing solution with buffered pH comprised between 4 and 8 according to anyone of claims 1-11, comprising a step of mixing said first component and said second component.

14. The method according to claim 13, further comprising a preparation step suitable for preparing the first component and selected among the following methods:

   - diluting, preferably in water, a concentrated solution of hydrogen peroxide, and
   - dissolving, preferably in water, at least one agent selected among urea peroxide, percarbonates, perborates, persulfates and tert-butyl hydroperoxide,
   wherein said preparation step precedes the step of mixing the first and second components.


**Patentansprüche**

1. Eine Desinfektions- und Kaltsterilisationszusammensetzung, die einen gepufferten pH-Wert zwischen 4 und 8 hat und mindestens eine erste Komponente und eine zweite Komponente umfasst,
worin die erste Komponente Wasserstoffperoxid umfasst und
die zweite Komponente Glukosepentaacetat und ein Lösungsmittel umfasst, worin das Lösungsmittel gewählt ist aus der Gruppe bestehend aus Aceton, einem Ester einer organischen Mono- oder Polycarbonsäure, Dimethylcarbonat und Mischungen davon.

2. Die Zusammensetzung gemäß Anspruch 1, worin die zweite Komponente weiter ein zweites Lösungsmittel umfasst, das gewählt ist aus der Gruppe bestehend aus Propylenglykolmonomethylether, Dipropylenglykolmonomethylether, Propylencarbonat und Mischungen davon.

3. Die Zusammensetzung gemäß Anspruch 1, worin die erste Komponente, die Wasserstoffperoxid umfasst, in Form einer wässerigen Lösung oder in fester Form vorliegt.

4. Die Zusammensetzung gemäß Anspruch 1, worin die erste Komponente Wasserstoffperoxid in einer Menge von 0,5 bis 35 Gewichtsprozent der ersten Komponente umfasst.

5. Die Zusammensetzung gemäß Anspruch 1, worin die zweite Komponente Glukosepentaacetat in einer Menge von 2 bis 30 Gewichtsprozent der zweiten Komponente und das Lösungsmittel in einer Menge von bis zu 98 Gewichtsprozent der zweiten Komponente umfasst.

6. Die Zusammensetzung gemäß Anspruch 1, worin die zweite Komponente wasserfrei ist.

7. Die Zusammensetzung gemäß Anspruch 1, worin die erste Komponente weiter mindestens ein Puffersystem umfasst.

8. Die Zusammensetzung gemäß Anspruch 1, worin die erste Komponente weiter mindestens ein Stabilisatorsystem umfasst.

9. Die Zusammensetzung gemäß Anspruch 8, worin das mindestens eine Stabilisatorsystem einen Stabilisator umfasst, der gewählt ist aus der Gruppe bestehend aus einer Diphosphonsäure, Ethylendiamintetraessigsäure - ethylenediaminetetraacetic acid, EDTA, - Natriumpyrophosphat und einem Natriumsalz einer Aminoalkylphosphonatsäure.

10. Die Zusammensetzung gemäß Anspruch 1, worin die erste Komponente weiter mindestens ein korrosionshemmendes System umfasst.

11. Die Zusammensetzung gemäß Anspruch 1, worin die erste Komponente weiter mindestens ein grenzflächenaktives System umfasst.

12. Ein Kit zur Herstellung einer Desinfektions- und Kaltsterilisationszusammensetzung, die einen gepufferten pH-Wert zwischen 4 und 8 gemäß einem beliebigen der Ansprüche 1-11 hat, wobei das Kit zwei physisch getrennte und nicht verbundene Fächer hat, worin

   - ein erstes Fach der Fächer die erste Komponente umfasst; und
   - ein zweites Fach der Fächer die zweite Komponente umfasst.

13. Ein Verfahren zur Herstellung einer Desinfektions- und Kaltsterilisationslösung mit gepuffertem pH-Wert zwischen 4 und 8 gemäß einem beliebigen der Ansprüche 1-11, das einen Schritt des Mischens der ersten Komponente und der zweiten Komponente umfasst.

14. Das Verfahren gemäß Anspruch 13, das weiter einen Herstellungsschritt umfasst, der zur Herstellung der ersten Komponente geeignet und aus folgenden Verfahren ausgewählt ist:

   - dem Verdünnen einer konzentrierten Lösung von Wasserstoffperoxid, vorzugsweise in Wasser, und
   - dem Auflösen mindestens eines Stoffs, gewählt aus Harnstoffperoxid, Percarbonaten, Perboraten, Persulfaten und tert-Butylhydroperoxid, vorzugsweise in Wasser,
   worin der Herstellungsschritt dem Schritt des Mischens der ersten und der zweiten Komponente vorangeht.

**Revendications**

1. Composition de désinfection et de stérilisation à froid, ayant un pH tamponné compris entre 4 et 8, et comportant au moins un premier composant et un second composant,
dans laquelle le premier composant comporte du peroxyde d'hydrogène et le second composant comporte du pentaacétate de glucose et un solvant,
dans laquelle le solvant est choisi parmi le groupe constitué d'acétone, d'un ester d'un acide organique mono- ou poly-carboxylique, du carbonate de diméthyle et leurs mélanges.

**2.** Composition selon la revendication 1, dans laquelle le second composant comporte en outre un second solvant choisi parmi le groupe constitué d'éther monométhylique de propylène glycol, d'éther monométhylique de dipropylène glycol, de carbonate de propylène et leurs mélanges de ceux-ci.

**3.** Composition selon la revendication 1, dans laquelle le premier composant comportant du peroxyde d'hydrogène se présente sous la forme d'une solution aqueuse ou sous une forme solide.

**4.** Composition selon la revendication 1, dans laquelle le premier composant comporte du peroxyde d'hydrogène en une quantité de 0,5 % à 35 % en poids sur le poids du premier composant.

**5.** Composition selon la revendication 1, dans laquelle le second composant comporte du penta-acétate de glucose en une quantité de 2 % à 30 % en poids sur le poids du second composant, et le solvant en une quantité jusqu'à 98 % en poids sur le poids du second composant.

**6.** Composition selon la revendication 1, dans laquelle le second composant est anhydre.

**7.** Composition selon la revendication 1, dans laquelle le premier composant comporte en outre au moins un système de tampon.

**8.** Composition selon la revendication 1, dans laquelle le premier composant comporte en outre au moins un système de stabilisation.

**9.** Composition selon la revendication 8, dans laquelle le au moins un système de stabilisation comporte un agent stabilisant choisi parmi le groupe constitué d'un acide diphosphonique, d'un acide éthylène diamine tétra-acétique (EDTA), d'un pyrophosphate de sodium et d'un sel de sodium d'un acide aminé phosphonate d'alkyle.

**10.** Composition selon la revendication 1, dans laquelle le premier composant comporte en outre au moins un système inhibiteur de corrosion.

**11.** Composition selon la revendication 1, dans laquelle le premier composant comporte en outre au moins un système de tensio-actif.

**12.** Kit pour préparer une composition de désinfection et de stérilisation à froid ayant un pH tamponné compris entre 4 et 8 selon l'une quelconque des revendications 1 à 11, ledit kit comportant deux compartiments physiquement séparés et non reliés, dans lequel :

- un premier compartiment desdits compartiments comporte ledit premier composant, et
- un second compartiment desdits compartiments comporte ledit second composant.

**13.** Procédé pour préparer une solution de désinfection et de stérilisation à froid avec un pH tamponné compris entre 4 et 8 selon l'une quelconque des revendications 1 à 11, comportant une étape de mélange dudit premier composant et dudit second composant.

**14.** Procédé selon la revendication 13, comportant en outre une étape de préparation adaptée pour préparer le premier composant et choisie parmi les procédés suivants :

- diluer, de préférence dans de l'eau, une solution concentrée de peroxyde hydrogène, et
- dissoudre, de préférence dans de l'eau, au moins un agent choisi parmi du peroxyde d'urée, des percarbonates, des perborates, des persulfates et de l'hydroperoxyde de tert-butyle,
dans lequel ladite étape de préparation précède l'étape de mélange des premier et second composants.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 953283 A
- IT 1334939
- EP 1371643 A
- US 3979313 A
- DE 3615787
- EP 2180786 A